(19) Europäisches Patentamt
European Patent Office
Office européen des brevets

(11) **EP 4 559 374 A1**

(12) **EUROPÄISCHE PATENTANMELDUNG**

(43) Veröffentlichungstag:
**28.05.2025 Patentblatt 2025/22**

(21) Anmeldenummer: **24210937.9**

(22) Anmeldetag: **05.11.2024**

(51) Internationale Patentklassifikation (IPC):
**A61B 1/00** *(2006.01)* **A61B 1/05** *(2006.01)*
**A61B 1/06** *(2006.01)* **A61B 5/00** *(2006.01)*

(52) Gemeinsame Patentklassifikation (CPC):
**A61B 1/00096; A61B 1/05; A61B 1/0638;**
**A61B 5/0075; A61B 5/0084;** A61B 1/00181;
A61B 1/046

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **24.11.2023 EP 23211973**

(71) Anmelder: **Erbe Elektromedizin GmbH**
**72072 Tübingen (DE)**

(72) Erfinder:
• **JAEGER, Jan**
**79100 Freiburg im Breisgau (DE)**
• **MOHAMMADI, Reza**
**72770 Reutlingen (DE)**

(74) Vertreter: **Rüger Abel Patentanwälte PartGmbB**
**Webergasse 3**
**73728 Esslingen a. N. (DE)**

(54) **ENDOSKOP MIT SENSOR ZUR BILDAUFNAHME UND GEWEBEUNTERSUCHUNG**

(57) Die Erfindung richtet sich auf ein Endoskop (10), das sowohl zur Bildgebung als auch zur Gewebeanalyse eingerichtet ist. Dazu weist es einen Bildsensor (23) auf, dem mehrere optische Einrichtungen (24, 25, 26) zugeordnet sind, um mit ein und demselben Bildsensor (23) verschiedene Aufgaben, insbesondere die Aufgabe der Bildgebung und Messaufgaben zeitgleich oder zeitlich gestaffelt auszuführen.

Fig. 1

EP 4 559 374 A1

**Beschreibung**

Die Erfindung betrifft ein Endoskop zur medizinischen Untersuchung und/oder Behandlung eines menschlichen oder tierischen Körpers bzw. Gewebeabschnitten desselben.

Zur Untersuchung und Behandlung von menschlichen oder tierischen Patienten sind Endoskope bekannt, die über einen länglichen Schaft verfügen, der in den Körper des Patienten einführbar ist.

Zum Beispiel offenbart die EP 2 075 617 A1 ein Endoskop mit einer optischen Einrichtung zur Spektraluntersuchung des vor dem distalen Ende des Endoskops liegenden Gewebes. Das Endoskop weist dazu an seinem distalen Ende ein Durchgangsloch auf, das als Lichteintrittsfenster dient. Mittels piezoelektrischer Elemente kann ein variabler Filter so beeinflusst werden, dass die von ihm durchgelassene Wellenlänge des Lichts verändert wird. Zu dem Endoskop gehört außerdem eine Beleuchtungseinrichtung und eine Bildaufnahmeeinrichtung, die in dem Endoskopgehäuse angeordnet sind. Das Endoskop ermöglicht somit eine spektrale Analyse des aufgenommenen Lichts.

Ein Verfahren zur Gewebebestimmung auf Basis einer spektrometrischen Messung offenbart dazu die EP 2 725 967 B1.

Außerdem offenbart der Fachartikel "3D-printed miniature spectrometer for the visible range with a 100 x 100 $\mu$m$^2$ footprint, Andrea Toulouse, Johannes Drozella, Simon Thiele, Harald Giessen und Alois Herkommer, https://doi.org/10.37188/lam.2021.002 eine miniaturisierte spektroskopische Messeinrichtung.

Ebenso offenbart der Fachartikel "Integrated spectroscopic analysis system with low vertical height for measuring liquid or solid assays" Yuhang Wan, Saoud A. Al-Mulla, Wang Peng, Kenneth D. Long, Benjamin A. Kesler, Patrick Su, John M. Dallesasse und Brian T. Cunnigham, http://www.elsevier.com/open-access/user license/1.0/ eine spektroskopische Messeinrichtung.

Eine weitere Bauart einer spektroskopischen Messeinrichtung geht aus der EP 2 284 509 A1 hervor.

Aus der WO 2018/072806 A1 ist eine Multifokallinsenanordnung bekannt, die direkt auf einen Bildsensor aufgebracht ist. Dieser Sensor eignet sich zur Aufnahme von Bildern eines Objekts unter gleichzeitiger Nutzung unterschiedlicher Brennweiten.

Die EP 0 939 894 B1 offenbart ein Miniaturspektrometer mit einer elektrischen Trägerplatte, auf der sowohl eine Lichtquelle als auch ein Lichtaufnahmeelement angeordnet sind. Diese Anordnung ist in eine durchsichtige Masse eingebettet, an der zwei Linsen unterschiedlichen Typs ausgeformt sind.

Die WO 2010/129324 offenbart ein Endoskop mit Arbeitskanal und am distalen Ende angeordneter durchsichtiger Endkappe. Durch diese hindurch kann ein Operationsfeld mittels einer LED-Lichtquelle beleuchtet werden. Außerdem enthält die Endkappe des Endoskops mehrere optische Komponenten, wie zum Beispiel eine optische Faser oder einen Bildsensor nebst zugehöriger Linse.

Die US 2005/0154277 A1 offenbart ebenfalls ein System mit mehreren Linsen, denen jedoch mehrere Bildsensoren zugeordnet sind. Diese optische Einrichtung kann Teil einer von einem Patienten verschluckbaren Kapsel zur Untersuchung des Gastrointestinaltrakts sein.

Die US 2009/0147076 A1 offenbart ein Endoskop mit einer am distalen Ende angeordneten optischen Einrichtung zur Aufnahme von Bildern aus mehreren Richtungen, einem rechteckigen Bildsensor am proximalen Ende des Endoskops. Die von den verschiedenen Linsen herrührenden Bilder werden auf unterschiedliche Zonen des Bildsensors projiziert.

Die US 2010/0141380 A1 offenbart ein Authentifizierungsverfahren und einen dazu passenden Fingerabdrucksensor. Dieser weist ein optisches Fenster auf, das eine ebene Oberseite zur Auflage eines Fingers und eine Rückseite aufweist, die ein Sägezahnprofil aufweist. Die Elemente des Sägezahnprofils bilden eine Serie von Prismen. Zur Beleuchtung der Rückseite dient eine Lichtquelle. In Nachbarschaft zu dieser sind eine erste Linse zur Erzeugung eines Abbilds auf einem CCD-Sensor sowie eine zweite Linse mit einem Diffraktionsgitter vorgesehen, das auf dem gleichen Sensor im Spektrum des vom Finger reflektierten Lichts abbildet. Der Sensor weist eine erste Zone auf, auf die ein Bild des Fingers projiziert wird sowie eine zweite Zone, auf die das Spektrum projiziert wird.

Es ist Aufgabe der Erfindung, ein diagnostisch einsetzbares Endoskop zu schaffen.

Diese Aufgabe wird mit dem Endoskop zur medizinischen Untersuchung und/oder Behandlung eines menschlichen oder tierischen Körpers gemäß Anspruch 1 gelöst:

Das erfindungsgemäße Endoskop weist an seinem distalen Ende eine Lichtaufnahmeeinrichtung und eine Beleuchtungseinrichtung auf. Zu der Lichtaufnahmeeinrichtung gehört mindestens eine abbildende erste optische Einrichtung, die zur Aufnahme eines Videostreams oder auch einzelner Fotos eingerichtet ist. Zu der Lichtaufnahmeeinrichtung gehört ein Bildsensor, insbesondere in Gestalt eines rechteckigen Kamerachips. Insbesondere eignet sich ein Bildsensor in MOS- oder CMOS-Technologie.

Die erste optische Einrichtung umfasst ein aus mindestens einem optischen Element, beispielweise einer Linse bestehendes Objektiv, das dazu eingerichtet ist, ein Kamerabild auf eine erste Zone des Bildsensors zu projizieren. So ist der ersten optischen Einrichtung eine erste Zone des Bildsensors zugeordnet. Die Zone kann eine eckige oder insbesondere auch eine runde, zum Beispiel kreisförmige, Form aufweisen. Die erste Zone ist eine Bildaufnahmezone. Der Bildsensor kann insbesondere ein rechteckiger Bildsensor sein. Sein Seitenverhältnis kann z.B. 16:9 sein. Die erste Zone kann auch rechteckig sein, dabei aber ein davon abweichendes Seitenverhältnis, z.B. 4:3 oder 1:1 oder anderes haben. Auch kann die erste Zone eine von der Rechteckform abweichende Form haben und z.B. Mehreckig oder rund sein.

Die Lichtaufnahmeeinrichtung umfasst außerdem eine zweite optische Einrichtung, der eine zweite Zone des Bildsensors zugeordnet ist. Diese zweite Zone kann Messzwecken dienen, womit sie eine Messzone bildet. Die Messzone und die erste Zone (Bildgebungszone) sind auf dem Bildsensor nebeneinander angeordnet. Sie können unterschiedliche Formen aufweisen. Während die Bildgebungszone beispielsweise rund sein kann, kann die Messzone eckig ausgebildet sein und insbesondere Rand- und Eckenbereiche des Bildsensors nutzen.

Das erfindungsgemäße Endoskop enthält somit an seinem distalen Ende eine erste als Bildaufnahmeeinrichtung dienende optische Einrichtung sowie eine zweite als Messeinrichtung dienende optische Einrichtung. Beide optische Einrichtungen verfügen vorzugsweise über unterschiedliche Lichteintrittsfenster und dort angeordnete optische Mittel, wie beispielsweise Objektive in Gestalt von Linsen, Lichtdurchtrittsspalten, optischen Gittern, Lichtfiltern und dergleichen. Beiden optischen Einrichtungen ist ein einziger Bildsensor gemeinsam zugeordnet, wobei zu der ersten und der zweiten optischen Einrichtung unterschiedliche Zonen des Bildsensors gehören.

Die beiden optischen Einrichtungen sind vorzugsweise jeweils eine optische Achse festlegend ausgebildet, wobei diese beiden optischen Achsen außerdem vorzugsweise parallel zueinander ausgerichtet sind. Alternativ können sie in einer in distaler Richtung von dem distalen Ende des Endoskops weg konvergierenden Ausrichtung zueinander orientiert sein. Durch diese Maßnahmen gelingt es, die bildgebende optische Einrichtung und die messende optische Einrichtung auf den gleichen Gewebebereich auszurichten. Auf diese Weise kann eine Messung von Gewebeeigenschaften zeitgleich mit der Beobachtung des Gewebes stattfinden, das beispielsweise ein Operationsgebiet festlegt.

Die zweite zu Messzwecken dienende optische Einrichtung kann zwei voneinander distanzierte Lichteintrittsfenster aufweisen, denen unterschiedliche zweite Teilzonen des Bildsensors zugeordnet sind. Vorzugsweise sind die beiden Lichteintrittsfenster der zweiten optischen Einrichtung an einer Seite der ersten optischen Einrichtung angeordnet, während das Lichtaustrittsfenster der zugeordneten Beleuchtungseinrichtung an der anderen Seite der ersten optischen Einrichtung angeordnet ist.

Die Lichtaufnahmeeinrichtung kann eine dritte optische Einrichtung aufweisen, der auf dem Bildsensor wenigstens eine dritte Zone zugeordnet ist. Die dritte optische Einrichtung kann zur Durchführung einer Messaufgabe eingerichtet sein, die sich von der Messaufgabe der zweiten optischen Einrichtung unterscheidet.

Die dritte optische Einrichtung kann ein eigenes Lichteintrittsfenster mit einem geeigneten Objektiv oder anderen optischen Mitteln aufweisen und für einen gewünschten Messzweck hergerichtet sein. Beispielsweise kann es sich bei der dritten optischen Einrichtung um eine Einrichtung zur Durchführung von Gewebeuntersuchungen handeln.

Die zweite und/oder dritte optische Einrichtung kann zur Durchführung der der diffusen Reflexionsspektroskopie eingerichtet sein. Zur Durchführung der Messung wird das Instrument mit seinem distalen Ende und insbesondere seinem Lichtaustrittsfenster in Gewebekontakt gebracht. Dabei können unterschiedlich weit von der Lichtquelle entfernte Teilzonen des Bildsensors Sensorbereiche unterschiedlich tief liegendes Gewebe vermessen. Die Lichtquelle kann schmalbandig oder breitbandig leuchtend ausgebildet sein. Das emittierte Licht kann ganz oder teilweise im Bereich sichtbaren Lichts oder ganz oder teilweise im Bereich von Infrarotlicht liegen. Es kann Information aus und über tiefer gelegene Gewebeschichten gewonnen werden.

Die zweite und/oder dritte optische Einrichtung kann alternativ zur Durchführung von Streulichtmessungen eingerichtet sein. Bei dieser werden mit der im Abstand zu dem Gewebe positionierten Sonde unterschiedlich weit von einer Lichtquelleweg liegende Gewebepartien unterschiedlich hell beleuchtet, so dass eine differenzielle Messung des von dem Gewebe gestreuten Lichts möglich wird. Dabei wird mit Abstand zum Gewebe Licht eingestrahlt und, je nachdem wie stark die Oberfläche streut, können in unterschiedlichen Abständen zur Lichtquelle unterschiedliche Intensitäten gemessen werden.

Die dritte zu Messzwecken dienende optische Einrichtung kann dazu zwei voneinander distanzierte Lichteintrittsfenster aufweisen, denen unterschiedliche dritte Teilzonen des Bildsensors zugeordnet sind. Vorzugsweise sind die beiden Lichteintrittsfenster der dritten optischen Einrichtung an zwei verschiedenen Seiten der ersten optischen Einrichtung angeordnet. Hingegen ist das Lichtaustrittsfenster der zugeordneten Beleuchtungseinrichtung an lediglich einer Seite der ersten optischen Einrichtung angeordnet. Die beiden unterschiedlichen dritten Zonen des Bildsensors sind in einem Abstand zueinander angeordnet. Beispielsweise können sie jeweils benachbart zu gegenüberliegenden Rändern des rechteckigen Bildsensors angeordnet sein. Außerdem können die beiden Lichteintrittsfenster der dritten optischen Einrichtung in unterschiedlichen Abständen zu einem Lichtaustrittsfenster der Beleuchtungseinrichtung angeordnet sein. Dies ermöglicht die Durchführung differentieller Messungen von Gewebeeigenschaften.

Eine weitere optische Einrichtung, der eine weitere Zone des Bildsensors zugeordnet sein kann, kann für ein weiteres Messverfahren reserviert sein. Insbesondere kann diese weitere optische Einrichtung zur Durchführung von Mikroskopie oder von mikroskopischen Messverfahren eingerichtet sein. Alternativen sind möglich. Zum Beispiel kann die Einrichtung zur Distanzmessung oder auch für anderweitige Messaufgaben eingerichtet sein.

Die Beleuchtungseinrichtung umfasst wenigstens eine Lichtquelle mit einer Lichtaustrittsrichtung, die vorzugsweise im Wesentlichen parallel zu der optischen Achse der ersten (bildgebenden) optischen Einrichtung festgelegt ist. Die Lichtquelle kann sowohl zur Bildaufnahme als auch zur Durchführung von Gewebemessungen dienen. Es kann vorgesehen werden, dass die Lichtquelle einen größeren Abstrahlwinkel hat als die aufnehmenden Optiken.

Die Lichtquelle kann eine Bandbreite aufweisen, die wenigstens so groß ist, wie die Bandbreite des Bildsensors hinsichtlich seiner Lichtempfindlichkeit. Es ist jedoch auch möglich, eine oder mehrere Lichtquellen vorzusehen, die jeweils lediglich Licht mit einer Bandbreite aussenden, die geringer ist als die Empfindlichkeitsbandbreite des Bildsensors und somit lediglich einen Ausschnitt derselben bedient. Diese Lichtquellen können je nach Messaufgabe ein- und ausschaltbar ausgestaltet sein, um zeitlich nacheinander oder auch gleichzeitig unterschiedliche Messaufgaben zu erfüllen. Die Lichtquelle kann insbesondere sowohl sichtbares Licht als auch infrarotes Licht emittierend ausgebildet sein. Dieser Spektralbereich kann auf eine einzige Lichtquelle oder auch auf mehrere Lichtquellen aufgeteilt sein.

Vorzugsweise ist die Bildgebungszone zwischen zwei Messzonen angeordnet, die zum Beispiel zu der zweiten optischen Einrichtung gehören. Diese zweite optische Einrichtung kann als Spektrometer ausgebildet sein. Ebenso kann bei Bedarf eine dritte optische Einrichtung vorgesehen sein, die ein Spektrometer oder auch eine differenzoptische Messeinrichtung ist. Durch unterschiedliche räumliche Anordnung oder auch die unterschiedliche Ausbildung hinsichtlich der spektralen Empfindlichkeiten der verschiedenen optischen Einrichtungen sind mit diesen unterschiedliche Messaufgaben lösbar. Beispielsweise kann die dritte optische Einrichtung zur Durchführung der diffusen Reflexionsspektroskopie eingerichtet sein. Dazu kann die dritte optische Einrichtung zur spektralen Filterung des empfangenen Lichts mehrere nebeneinander angeordnete Filter oder ein Filter mit ortsabhängiger Farbe aufweisen, welches das empfangene Lichtsignal, beispielsweise in dem Wellenlängenbereich von 600 nm bis 900 nm spektral filtert. Die dazu verwendeten Filter können unmittelbar auf den Bildsensor aufgebracht sein. Alternativ können ein oder mehrere diffraktive optische Elemente, wie z.B. ein Prisma oder Gitter in einem Abstand zu dem Sensor vorgesehen sein.

Außerdem kann die Beleuchtungseinrichtung Elemente zur strukturierten Beleuchtung des Gewebes, beispielsweise in Gestalt eines Lichtgitters oder eines anderen Musters, eingerichtet sein. Die Beleuchtungseinrichtung kann dazu eine Laserdiode, insbesondere ein Infrarotlaserdiode aufweisen, der ein diffraktives optisches Element zugeordnet ist, um ein Lichtmuster auf das Gewebe zu projizieren. Geschieht dies im Infrarotbereich, ist das Lichtmuster für den Anwender nicht sichtbar. Jedoch kann auf diese Weise ein 3D-Modell der Gewebeoberfläche erzeugt werden.

Es ist möglich, ein und dieselbe Zone des Bildsensors zwei verschiedenen optischen Einrichtungen zuzuordnen. Zum Beispiel kann der Bildgebungsbereich (erste Zone) des Sensors gleichzeitig sowohl zur Bildgebung mittels sichtbaren Lichts als auch zur 3D-Modellbildung mittels infraroten strukturierten Lichts genutzt werden. Auch andere Bereiche des Bildsensors können mehrfach genutzt werden.

Der Bildsensor ist vorzugsweise ein RGB-IR-Sensor. Ein solcher enthält über seine rechteckige Lichtaufnahmefläche verteilt in einem Raster angeordnete Lichtaufnahmeelemente (Pixel). Vorzugsweise sind dabei Lichtaufnahmeelemente für blaues, grünes, rotes und infrarotes Licht in geeigneter Weise miteinander abwechselnd über die Fläche des Sensors verteilt. Dazu kann auf dem eigentlichen Bildsensor herstellerseits bereits eine Farbfilter-Matrix (Bayer-Matrix) zur Farbdarstellung aufgebracht sein. Die Anordnung der einzelnen Lichtaufnahmeelemente ist vorzugsweise in allen Zonen des Bildsensors, d.h. sowohl in der Bildaufnahmezone als auch in der Messzone gleich. Die Farbfiltermatrix kann sich sowohl über die zur Bildgebung dienende erste Zone als auch über die zu Messzwecken dienende zweite oder dritte Zone erstrecken. Alternativ kann die Farbfilter-Matrix in der zweiten und/oder dritten Zone auch fehlen oder, wenn sie herstellerseits vorgesehen war, auch nachträglich entfernt worden sein.

Der Bildsensor kann Pixel aufweisen, die für zwei, drei, vier oder auch mehrere verschiedene Lichtwellenlängen (d.h. Farben) empfindlich sind und damit Licht mit einer dementsprechenden spektralen Auflösung analysieren. Bei einem Bildsensor mit vier verschiedenfarbig empfindlichen Pixeln können somit vier verschiedene Spektrallinien (d.h. Lichtbestandteile) des empfangenen Lichts unterschieden werden.

Es ist aber darüber hinaus möglich, mit dem gleichen Bildsensor eine Anzahl von Spektrallinien zu erfassen, die deutlich größer ist, als die Anzahl der von dem Bildsensor selbst unterschiedenen Farben. Dies kann insbesondere mit Multibandpassfiltern erfolgen, die jeweils Licht mit unterschiedlichen Farben (d.h. Licht in mehreren unterschiedlichen Längenwellenbereichen) passieren lassen und ansonsten absorbieren oder reflektieren. Vorzugsweise können dazu zwei oder mehrere voneinander hinsichtlich ihrer Farben unterschiedliche Multibandpassfilter Verwendung finden, die jeweils eine größere Gruppe von Pixeln überdeckend und in Nachbarschaft zueinander direkt oder mit Abstand nebeneinander im Lichtweg, z.B. direkt auf dem Bildsensor angeordnet sind.

Die verschiedenfarbigen Bestandteile des von dem Multibandpassfilter durchgelassenen Lichts treffen die verschiedenfarbigen Pixel des Bildsensors gleichermaßen. Die Pixel sind für verschiedene, z.B. vier, verschiedene Wellenlängenbereiche (Farbbereiche) empfindlich (Empfindlichkeitsbereiche). Die Empfindlichkeitsbereiche können sich überschneiden. Deswegen kann es sein, dass durchgelassenes Licht einer einzigen Farbe zwei wellenlängenmäßig benachbarte Pixeltypen des Bildsensors anspricht. Auch kann es passieren, dass in einem Empfindlichkeitsbereich eines Bildsensortyps zwei oder mehrere Durchlassbereiche der Multibandpassfilter liegen. Fallen mehrere Durchlassbereiche des Multibandpassfilters in ein und denselben Empfindlichkeitsbereich, lässt sich das von dem gleichen Pixeltyp empfangenen Licht sich rechnerisch dann besonders einfach verschiedenen Spektrallinien zuordnen, wenn in dem Empfindlichkeitsbereich nur ein und in dem benachbarten überschneidenden Empfindlichkeitsbereich kein Durchlassbereich des gleichen Multibandpassfilters liegt.

Weitere Einzelheiten vorteilhafter Weiterbildungen und Details der Erfindung ergeben sich aus der Zeichnung, der Beschreibung und Ansprüchen. In der Zeichnung zeigen:

Figur 1 ein erfindungsgemäßes Endoskop, in schematischer Darstellung,

Figur 2 eine Stirnansicht des Endoskops nach Figur 1, in schematisierter Darstellung,

Figur 3 den Bildsensor und die Beleuchtungseinrichtung des Endoskops nach Figur 1 und 2, in schematisierter Draufsicht,

Figur 4 eine Pixelanordnung des Bildsensors des Endoskops,

Figur 5 die optischen Verhältnisse bei der Durchführung einer Gewebeuntersuchung mit auf das Gewebe aufgesetzter Endoskopspitze,

Figur 6 das Endoskop in bildgebendem Einsatz in einer von dem Gewebe distanzierter Position

*Figur 7 den Bildsensor des Endoskops nach Figur 1 und 3 mit Bildgebungszone und Filtern zur Ausbildung eines einem multi-Kanal Spektrometers,*

*Figur 8 den Bildsensor nach Figur 7 ohne Filter,*

Figur 9 ein erstes Diagramm zur Veranschaulichung von Durchlassbereichen eines ersten Filters und Empfindlichkeitsbereichen des Bildsensors,

Figur 10 ein zweites Diagramm zur Veranschaulichung von zweiten Durchlassbereichen eines Filters und Empfindlichkeitsbereichen des Bildsensors,

Figur 11 ein Diagramm zur Veranschaulichung von mit dem Bildsensor erfassbaren Spektrallinien.

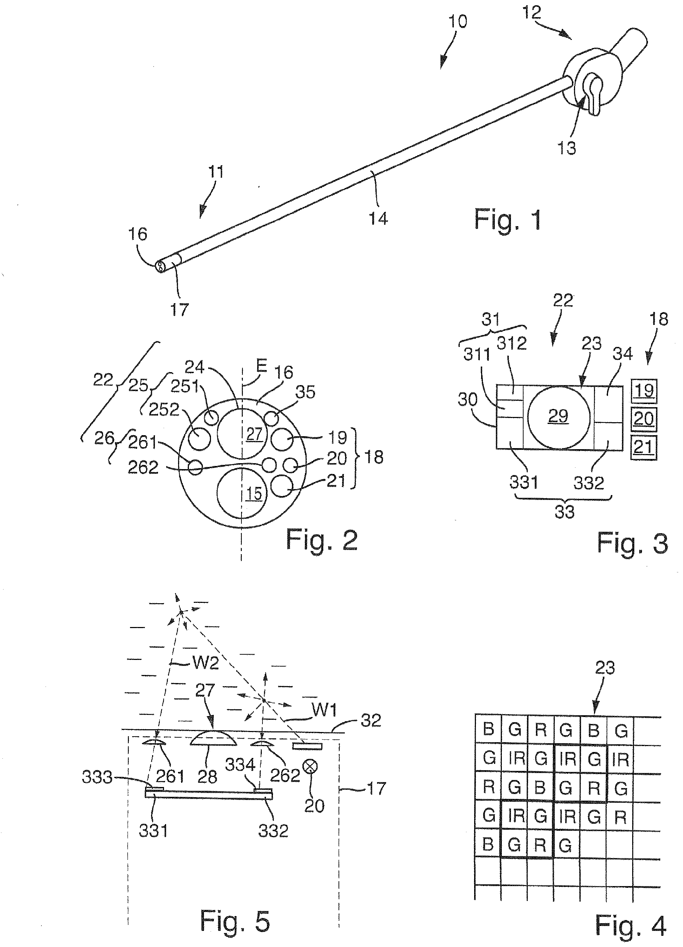

In Figur 1 ist ein erfindungsgemäßes Endoskop 10 in Übersichtsdarstellung veranschaulicht. Das Endoskop 10 weist ein distales Ende 11 auf, das bei Durchführung einer Operation in einen Patienten, beispielsweise in ein Körperlumen desselben, einzuführen ist. Das Endoskop 10 wird dann von seinem proximalen Ende 12 her gesteuert, an dem ein oder mehrere Bedienelemente 13 vorgesehen sein können. Je nach Ausbildung des Endoskops 10, können solche Bedienelemente 13, beispielsweise zum Bewegen, insbesondere zum Abwinkeln eines Abschnitts des distalen Endes 11 genutzt werden. Der längliche Schaft 14 des Endoskops kann im Übrigen steif oder bedarfsweise auch flexibel ausgebildet sein.

Das Endoskop kann einen oder mehrere Arbeitskanäle 15 aufweisen, der oder die sich bis zu der eben oder auch gewölbt ausgebildeten endseitigen Stirnfläche 16 des Schafts 14 erstrecken. Der oder die Arbeitskanäle 15 dienen der Aufnahme von nicht weiter veranschaulichten Sonden oder Instrumenten, die als Werkzeuge zur Einwirkung auf biologisches Gewebe dienen. Solche Instrumente können zum Beispiel HF-chirurgische Instrumente, kryochirurgische Instrumente oder andere zur Einwirkung auf Gewebe geeignete Werkzeuge sein.

Das erfindungsgemäße Endoskop 10 weist in seinem distalen Endabschnitt 17 eine umfangreiche optische Einrichtung 22 auf, zu deren Erläuterung nachfolgend auf die Figuren 2 bis 6 Bezug genommen wird. Diese optische Einrichtung 22 ist in dem distalen Endabschnitt 17 des distalen Endes 11 untergebracht, der unabhängig davon, ob der Schaft 14 steif oder flexibel ausgebildet ist, selbst formstabil und dabei im Wesentlichen zylindrisch ausgebildet ist. Die Stirnfläche 16 kann hingegen gewölbt und dabei beispielsweise halbkugelförmig ausgebildet sein. Andere Formgebungen, insbesondere eine im Wesentlichen ebene Ausbildung mit einer gerundeten Übergangszone zur zylindrischen Umfangsfläche, sind ebenfalls möglich und bestimmten nachfolgend erläuterten Messaufgaben dienlich.

Zu der in dem Endabschnitt 17 untergebrachten optischen Einrichtung gehört zunächst eine Beleuchtungseinrichtung 18, zu der mindestens eine, gegebenenfalls aber auch mehrere, zum Beispiel zwei oder drei Lichtquellen 19, 20, 21 gehören können, die in Figur 2 anhand ihrer Lichtaustrittsfenster veranschaulicht sind. Die Lichtquellen 19, 20, 21 können Lichtquellen mit gleichen optischen Eigenschaften, aber insbesondere auch Lichtquellen mit unterschiedlichen Eigenschaften sein. Die Unterschiede können sich auf:

- die Bandbreite und/oder

    - die spektrale Zusammensetzung des emittierten Lichts,

    - auf die räumliche Verteilung des Lichts, beispielsweise hinsichtlich

- der Öffnungswinkel oder

- erzeugter Lichtmuster, oder auch

- auf zeitliche Eigenschaften, wie beispielsweise

- Dauerlicht,

- intermittierendes Licht sowie die

- Zeitpunkte der Aktivierung der jeweiligen Lichtquellen beziehen.

Beispielsweise kann die erste Lichtquelle 19 eine Lichtquelle für den roten bis infraroten Längenwellenbereich, zum Beispiel von 600 nm bis 900 nm sein. Die zweite Lichtquelle 20 kann zum Beispiel eine Weißlichtquelle für den Wellenlängenbereichs des sichtbaren Lichts oder auch eine farbige im sichtbaren Bereich liegende Lichtquelle sein. Außerdem kann die zweite Lichtquelle eine sowohl den Bereich sichtbaren Lichts, wenigstens teilweise als auch den Infrarotbereich wenigstens teilweise umfassende Lichtquelle sein. Weiter kann die zweite Lichtquelle eine reine Infrarotlichtquelle sein. Die Lichtquelle 21 kann eine Weißlichtquelle sein. Sie kann darauf eingerichtet sein, den Operationsbereich gleichmäßig auszuleuchten. Alternativ kann sie darauf eingerichtet sein, lediglich einen Teilbereich auszuleuchten. Insbesondere kann sie als Projektionseinrichtung ausgebildet und darauf eingerichtet sein, ein Bild oder ein Lichtmuster zu erzeugen, um beispielsweise ein Linienbild, ein Karobild oder ein anderweitiges ruhendes oder bewegtes Bild auf das vor dem distalen Ende 11 des Endoskops liegende Gewebe zu projizieren.

Die Lichtquellen 19, 20, 21 können miteinander übereinstimmende Lichtaustrittsrichtungen (optische Achsen) oder auch unterschiedliche Lichtaustrittsrichtungen aufweisen. Bei einer bevorzugten Ausführungsform weist wenigstens eine der drei Lichtquellen 19, 20, 21 eine Lichtaustrittsrichtung auf, die mit der Längsrichtung des Schafts 14 übereinstimmt. Vorzugsweise sind die Lichtaustrittsrichtungen von zwei Lichtquellen oder auch alle drei Lichtquellen parallel zueinander orientiert. Die Öffnungswinkel der aus den Lichtquellen 19, 20, 21 austretenden Lichtkegel können gleich oder auch unterschiedlich bemessen sein. Dabei können ein oder mehrere Lichtquellen 19, 20, 21 jeweils einen kreiskegelförmigen Lichtaustritt mit kreisrunder Lichtauftrittsfläche aufweisen. Eine oder mehrere der Lichtquellen 19, 20, 21 können auch davon abweichende Lichtauftrittsflächen, beispielsweise ovale, halbkreisförmige oder rechteckige Lichtaustrittsflächen definieren.

Die Beleuchtungseinrichtung 18 ist Teil einer zur Bildaufnahme und zur Durchführung von Messungen an Gewebe geeigneten Sensoreinrichtung, zu der, neben der Beleuchtungseinrichtung 18, eine Lichtaufnahmeeinrichtung 22 gehört. Diese umfasst einen Bildsensor 23, der in Figur 3 gesondert veranschaulicht ist. Der Bildsensor 23 kann ein sogenannter Kamerachip sein, der eine Vielzahl in einem vorgegebenen Raster angeordneter lichtempfindlicher Zellen (sogenannter Pixel) aufweist. Die einzelnen Pixel sind typischerweise für jeweils unterschiedliche Lichtfarben empfindlich, beispielsweise für blau, grün und rot. Bei einer bevorzugten Ausführungsform sind außerdem infrarotempfindliche Pixel vorhanden. Entsprechend sind die Pixel in Figur 4 mit "B" für blaues, "G" für grünes, "R" für rotes und "IR" für infrarotes Licht gekennzeichnet. Es handelt sich um einen RGB-IR-Sensor.

Dem Bildsensor 23 sind mehrere optische Einrichtungen 24, 25, 26 zugeordnet, die in Figur 2 anhand ihrer an der Endfläche 16 angeordneten Lichteintrittsfenster veranschaulicht sind.

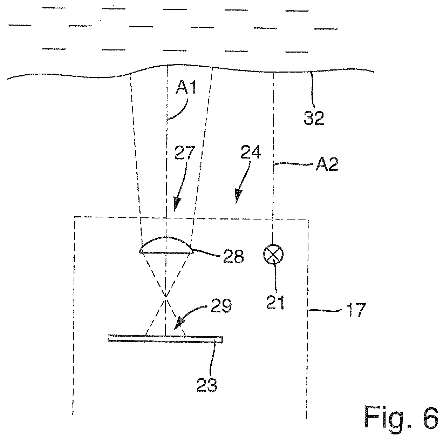

Die erste optische Einrichtung 24 ist eine bildgebende Einrichtung. Zu dieser gehört ein Objektiv 27, das in Figur 5 und 6 durch eine Linse 28 veranschaulicht ist. Das Objektiv 27 kann aber auch andere und/oder weitere optische Elemente aufweisen. Das Objektiv 27 ist ein bildgebendes Objektiv und dazu eingerichtet, ein Objektbild auf eine erste Zone 29 des Bildsensors 23 zu projizieren. Diese Zone 29 kann, wie Figur 3 veranschaulicht, eine von dem Umriss des Bildsensors 23 abweichende Form aufweisen, insbesondere rund, quadratisch oder rechteckig sein, z.B. mit einem Seitenverhältnis von 4:3. Vorzugsweise ist die Zone 29 etwa zentral auf dem Bildsensor 23 angeordnet.

Zwischen einer Schmalseite 30 des Bildsensors 23 und der Zone 29 kann eine zweite Zone 31 vorgesehen sein, die als Messzone dient. Diese zweite Zone 31 kann in mehrere, bspw. zwei Teilzonen 311 und 312 aufgeteilt sein, die zwischen der ersten Zone 29 und der Schmalseite 30 angeordnet sind. Während die erste Zone 29 eine Bildgebungszone ist, ist die zweite Zone 31 eine Messzone.

Der ersten optischen Einrichtung 24 ist mindestens eine der Lichtquellen 19, 20, 21 zugeordnet. Beispielsweise kann die Lichtquelle 21 dazu dienen, Licht zur Beleuchtung einer Gewebeoberfläche 32 abzustrahlen (Figur 6), wobei die erste optische Einrichtung 24 dann dazu dient, ein entsprechendes Bild aufzunehmen. Hingegen kann der zweiten optischen Einrichtung 25 die gleiche oder eine andere Lichtquelle, zum Beispiel die Lichtquellen 19, zugeordnet sein. Während die Lichtquelle 19 (oder 20 oder 21) auf einer Seite einer durch die erste optische Einrichtung 24 und den Arbeitskanal 15 gehende Mittellinie E angeordnet ist, ist die optische Einrichtung 25 vorzugsweise auf der anderen Seite dieser Linie E angeordnet. Die Einrichtung 25 kann ein oder zwei benachbart zueinander angeordnete Lichteintrittsfenster 251, 252 aufweisen, die Licht aufnehmen und auf die Teilzonen 311, 312 des Bildsensors 23 leiten.

Eine weitere Möglichkeit der Anordnung einer Lichtquelle, beispielsweise der Lichtquelle 20 und der Lichteintrittsfenster 261, 262 der dritten optischen Einrichtung zeigen Figur 2 und 3. Den Lichteintrittsfenstern 261, 262 der dritten optischen Einrichtung 26 ist eine dritte Zone 33 des Bildsensors 23 zugeordnet, die wiederum zwei Teilzonen 331, 332 umfassen kann. Vorzugsweise sind die beiden Teilzonen 331, 332 zu beiden Seiten der Bildgebungszone 29 angeordnet. Somit sind zu beiden Seiten der Bildgebungszone 29 Messzonen bzw. Teile derselben 331, 332 vorgesehen.

Außerdem kann in einem verbleibenden Abschnitt des Bildsensors 23 eine vierte Messzone 34 vorgesehen sein, der ebenfalls ein eigenes Objektiv 35 zugeordnet ist, das zum Beispiel der Mikroskopie dienen kann.

Auf dem rechteckigen Bildsensor 23 sind somit mindestens eine der Bildgebung dienende Zone 29 sowie mindestens eine, vorzugsweise mehrere, weitere der Messung von Gewebeeigenschaften dienende Zonen 31, 33, 34 vorgesehen. Während die Bildgebungszone 29 vorzugsweise symmetrisch, d.h. mittig auf dem Bildsensor 23 angeordnet ist, können die zur Durchführung von Messaufgaben geeigneten Zonen 31, 33, 34 des Bildsensors 23 zu beiden Seiten der Bildgebungszone 29 symmetrisch oder asymmetrisch angeordnet werden.

Der Bildsensor 23 ist vorzugsweise ein viele Pixel aufweisender Halbleitersensor, der sowohl für die Bildgebungszone 29 als auch für die Messzonen 31, 33, 34 den gleichen einheitlichen Aufbau aufweist.

Das insoweit beschriebene Endoskop 10 lässt sich vielseitig einsetzen:

In Figur 6 ist das Endoskop 10 bzw. sein Endabschnitt 17 bei Betrieb zur Bildgebung schematisch veranschaulicht. Es ist in Distanz zu einer Gewebeoberfläche 32 positioniert. Mittels der dritten Lichtquelle 21 wird die Gewebeoberfläche 32 beleuchtet. Über das Objektiv 27 wird ein entsprechendes Gewebebild auf dem Bildsensor 23 und dort insbesondere auf die Bildgebungszone 29 projiziert. Der Bildsensor 23 erzeugt somit Bilder bzw. einen Videostream, der über nicht weiter veranschaulichte Mittel zu dem proximalen Ende des Endoskops 12 und von dort zu einer Bildwiedergabeeinrichtung, beispielsweise einer VR-Brille, einem Bildschirm oder dergleichen, weitergeleitet werden kann.

Ist die Lichtquelle 21 eine Lichtquelle zur strukturierten Beleuchtung oder ist sie auf die Abgabe strukturiertem Licht umschaltbar, kann sie dazu genutzt werden, ein Lichtmuster auf die Gewebeoberfläche 32 zu projizieren. Das entstehende Bild kann einer Bildverarbeitungseinrichtung zugeleitet werden, die daraus ein 3D-Model der Gewebeoberfläche 32 errechnet und zur Wiedergabe bereitstellt.

Es ist möglich, die Lichtquelle 21 derart zu gestalten, dass sie im Bereich sichtbaren Lichts unstrukturiertes Licht und im Bereich infraroten (nicht sichtbaren Lichts) strukturiertes Licht abgibt. Auf diese Weise kann der Bildsensor 23 einerseits ein optisches Bild zur Wiedergabe für einen Chirurgen und andererseits ein Infrarotbild erzeugen, aus dem die Bildverarbeitungseinrichtung ein Reliefbild, d.h. ein 3D-Modell der Gewebeoberfläche 32 ermitteln und zur Weiterverarbeitung oder Betrachtung bereitstellen kann.

Figur 5 veranschaulicht einen Betriebsmodus, bei dem keine Bildgebung dafür aber eine Gewebemessung erfolgt. Dazu ist die distale Stirnfläche 16 des Endoskops 10 auf die Gewebeoberfläche 32 aufgesetzt. Der Betrieb wird am Beispiel der Lichtquelle 20 erläutert, die mit den beiden Teilen 261, 262 der optischen Einrichtung 26 zusammenwirkt. Beispielsweise sendet die Lichtquelle 20 breitbandiges Infrarotlicht aus, das in das Gewebe eindringt und dort gestreut wird. Das Licht gelangt zu den Lichteintrittsfenstern der beiden Teile 261, 262 auf unterschiedlich langen Wegen W1, W2. Infolge des kürzeren Weges W1 erhält die Teilzone 332 ein Lichtsignal, das in oberen oberflächennahen Gewebeschicht gestreut wurde. Wegen des längeren Weges W2 erhält die Teilzone 331 ein Lichtsignal, das in tieferen oberflächenfernen Gewebeschicht gestreut wurde. Damit lässt sich durch Vergleich der beiden Signale die Streuung des Gewebes, die Art der beleuchteten Gewebeschichten und so auch die Nähe zu einem Organ bestimmen. Die insbesondere, wenn das im Gewebe liegende Organ andere lichtstreuende Eigenschaften hat, als das einbettende umgebende Gewebe. Außerdem wird das Licht aufgrund der unterschiedlichen Lichtweglängen unterschiedlich stark absorbiert.Durch die räumlich differenzielle Messung ist es zusätzlich möglich, Streuung und Absorption zu differenzieren, sofern die vorliegende Gewebetyp eine homogene Struktur aufweist.

Zur Durchführung dieser differentiellen diffusen Reflexionsspektroskopie können alternativ Lichtquellen für vorzugsweise nicht zu schmalbandiges Infrarotlicht, oder für sichtbares farbiges oder weißes Licht oder für Licht vorgesehen werden, das sowohl Anteile im sichtbaren als auch im infraroten Spektralbereich enthält.

Zumindest eine der zu Messzwecken dienende Zonen 31, 33 oder zumindest eine Teilzone 311, 312, 331, 332 derselben kann zur Durchführung spektrometrischer Messaufgaben eingerichtet sein. Dazu können in den einzelnen zur Messung dienenden Zonen 31 und/oder 32 spektral filternde Elemente aufgebracht oder in Relation zu diesen angeordnet sein. Im einfachsten Fall werden schmalbandige Farbfilter 333, 334 (Figur 5), zum Beispiel interferenzoptische Filter, direkt auf den Bildsensor 23 aufgebracht, wobei jeweils einzelne Pixel oder Pixelgruppen mit einem Filter für eine ausgewählte Wellenlänge versehen sind. Auf diese Weise lässt sich mit dem Bildsensor 32 parallel zur Bildgebung eine Spektralanalyse des von der Gewebeoberfläche 32 rückgestreuten Lichts durchführen. Anstelle einzelner nebeneinander angeordneter Filter kann auch ein sogenanntes linear variables Filter vorgesehen werden, dessen Durchlasswellenlänge entlang einer Linie, vorzugsweise einer Geraden variiert.

Mit dem erfindungsgemäßen Endoskop 10 lassen sich verschiedene Messaufgaben gleichzeitig oder nacheinander durchführen. Ist das Endoskop 10 mit seiner Stirnfläche im Abstand zu der Gewebeoberfläche 23 positioniert, kann folgendes durchgeführt werden:

- Bildgebung mittels der ersten optischen Einrichtung 24

- 2D-Bildgebung mit sichtbarem Licht und gleichzeitige 3D-Erfassung mittels strukturierter Beleuchtung mit IR-Licht

- Bildgebung mittels der optischen Einrichtung 24 und differentielle diffuse Reflexionsspektroskopie mittels der dritten optischen Einrichtung 26

- Bildgebung mittels der optischen Einrichtung 24 und Mikroskopie mittels der vierten optischen Einrichtung 35

- Bildgebung mittels der optischen Einrichtung 24, diffuse Reflexionsspektroskopie mittels der dritten optischen Einrichtung 26 und Mikroskopie mittels der vierten optischen Einrichtung 35

Ist das Endoskop 10 hingegen mit seiner Stirnfläche auf die Gewebeoberfläche 23 aufgesetzt, kann folgendes durchgeführt werden:

- diffuse Reflexionsspektroskopie mittels der zweiten optischen Einrichtung 25 und/oder

- diffuse Reflexionsspektroskopie mittels der dritten optischen Einrichtung 26.

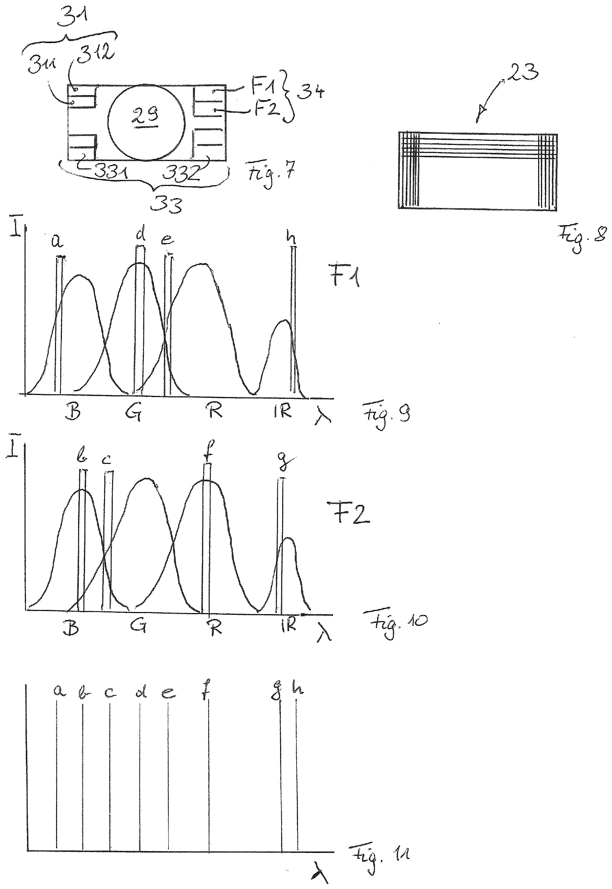

Wie erwähnt können die Zonen des Bildsensors 23, die anders als die Bildgebungszone 29 nicht zur Bildgebung genutzt werden, zu Messzwecken herangezogen werden. Dabei ist es möglich, mit dem Bildsensor 23 spektroskopische Messungen durchzuführen. Eine besonders vorteilhafte Möglichkeit dazu offenbaren die Figuren 7 bis 11, in der mindestens eine der Messzonen 31, 33, 34 als Spektrometer und dabei insbesondere als ein zur Analyse von diffusem Licht geeignetes Spektrometer ausgebildet ist.

Der Bildsensor 23 enthält mehrere für unterschiedliche Wellenlängenbereiche empfindliche Pixel B (blau), G (grün), R (rot), Infrarot (IR). Diese können nach dem Schema gemäß Figur 4 oder auch in einem anderen Schema angeordnet sein. Außerdem kann der Bildsensor 23 auch eine abweichende Anzahl von Pixeltypen, beispielsweise lediglich zwei oder drei, aufweisen. Die Empfindlichkeitsbereiche der einzelnen Pixel B, G, R, IR sind in Figuren 9 und 10 veranschaulicht. Wie ersichtlich haben alle Empfindlichkeitsbereiche jeweils ein Maximum, wobei die Lichtempfindlichkeit des jeweiligen Pixels B, G, R, I, R zu beiden Seiten des Maximums abfällt. Die Empfindlichkeitsbereiche benachbarter Pixel B/G, G/R können sich deutlich überlappen wie die Figuren 9 und 10 veranschaulichen.

Zu der spektrometrischen Messzone 34 gehören zwei Filter F1 und F2, die nicht überlappend angeordnet sind. Sie können direkt aneinandergrenzend oder auch in einem gewissen Abstand zueinander auf dem Bildsensor 23 angeordnet sein. Der Filter F1 ist ein erster Multibandpassfilter, der Licht durch die in Figur 9 idealisiert veranschaulichten Durchlassbänder a, d, e und h passieren lässt, Licht anderer Wellenlängen jedoch absorbiert oder reflektiert. In Figur 10 ist die Durchlasscharakteristik des zweiten Multibandpassfilters F2 veranschaulicht, der, wie dort idealisiert dargestellt ist, Licht der Durchlassbänder b, c, f und g passieren lässt, andere Wellenlängenbereiche jedoch sperrt oder absorbiert oder reflektiert.

Zur Lichtauswertung existieren nun zwei Pixelgruppen, nämlich die Gruppe der Pixel B, G, R, IR, die von Licht des Multibandpassfilters F1 beleuchtet werden und eine zweite Gruppe mit Pixeln B, G, R, IR, die von Licht des Multibandpassfilters F2 beleuchtet wird. Wie aus Figur 9 hervorgeht, kann dabei Licht z.B. des Durchlassbands e sowohl in den Pixeln G als auch in den Pixeln R der Gruppe des Filters F1 ein Signal hervorrufen. Entsprechend kann Licht des Durchlassbands c Signale in den Pixeln B, G der Gruppe unter dem Filter F2 Signale hervorrufen. Obwohl Licht aus beiden Durchlassbändern d und e die Pixel G grüner Empfindlichkeit ansprechen und obwohl Licht aus den Durchlassbändern b, c die Pixel B blauer Empfindlichkeit anspricht, lassen sich sowohl die Spektralanteile der Durchlassbänder d, e als auch die Spektralanteile der Durchlassbänder b, c voneinander separieren. Dies wird nachfolgend an dem Beispiel der Intensitäten I der jeweils im Überlappungsbereich liegenden Spektralanteile der Durchlassbänder c und e demonstriert: Besonders einfach ist die Trennung der Spektralanteile der Durchlassbänder d und e des Filters F1 deswegen, weil neben dem im Überlappungsbereich liegenden Durchlassband e nur ein einziges Durchlassband d liegt. Und weiter, weil in einem der benachbarten Empfindlichkeitsbereich der Pixel G und R kein Durchlassbereich des gleichen Filters F1 und in dem anderen der benachbarten Empfindlichkeitsbereiche der Pixel G und R kein Durchlassband liegt. Das Durchlassband f ist dem zweiten Filter F2 zugeordnet und kann dort unverfälscht an dem roten Pixel R abgelesen werden.

Entsprechend spricht Licht der Durchlassbänder b und c unter dem Filter F2 beide Pixel B und G an. Jedoch ist lediglich für die Pixel B ein weiteres Durchlassband b vorhanden. Die grünen Pixel G erhalten jedoch nur Licht aus dem Durchlassband c, während Licht aus dem Durchlassband d über die Pixel aus dem ersten Filterbereich F1 gewonnen wird.

Die Intensität I der Durchlassbänder c und e lässt sich wie folgt bestimmen:

Das Verhältnis der Signale der Pixel B und G im Überlappungsbereich bei der Wellenlänge des Durchlassbands c ist aus Datenblättern bekannt und wird hier $F_{zwischen\,GB\,an\,c}$ bezeichnet. Das von dem grünen Pixel G für das Durchlassband c gelieferte Signal $G_{an\,c}$ wird mit dem Faktor $F_{zwischen\,GB\,an\,c}$ multipliziert, um die Intensität von $B_{an\,c}$ zu erhalten. Die Signale oder Intensitäten

$I_c$ und $I_b$ können wie folgt berechnet werden:

$$B_{an\,c} = G \cdot F_{zwischen\,GB\,an\,c}$$

$$I_c = B_{an\,c} + G$$

$$I_b = B - B_{an\,c}$$

$$G_{an\,e} = R \cdot F_{zwischen\,RG\,an\,e}$$

$$e = G_{an\,e} + R$$

$$d = G - G_{an\,e}$$

Es wird damit das in Figur 11 dargestellte Spektrum mit 8 Spektrallinien erhalten. Auf weiteren Flächenbereichen des Bildsensors 23 können weitere Multibandpassfilter vorgesehen werden, um die spektrale Auflösung weiter zu verbessern. Die Bedingung dazu ist, dass die Verhältnisse der spektralen Sensivitäten wellenlängenmäßig benachbarter Pixel im Überlappungsbereich bekannt sind und lediglich eine Multibandpass-Doppelbelegung pro Kanal (RGB) vorliegt. Diese Doppelbelegungen sind in Figur 9 d und e und in Figur 10 b und c. Hilfreich ist, wenn in dem gleichen oder wenigstens einem der jeweils anderen Filter in dem benachbarten Farbkanal (hier R und G) jeweils keine Kanalbelegung (kein Durchlassbereich des entsprechenden Filters) vorhanden ist.

Die Erfindung richtet sich auf ein Endoskop 10, das sowohl zur Bildgebung als auch zur Gewebeanalyse eingerichtet ist. Dazu weist es einen Bildsensor 32 auf, dem mehrere optische Einrichtungen 24, 25, 26 zugeordnet sind, um mit ein und demselben Bildsensor 23 verschiedene Aufgaben, insbesondere die Aufgabe der Bildgebung und Messaufgaben zeitgleich oder zeitlich gestaffelt auszuführen.

Bezugszeichen:

| | |
|---|---|
| 10 | Endoskop |
| 11 | distales Ende des Endoskops 10 |
| 12 | proximales Ende des Endoskops 10 |
| 13 | Bedienelement des Endoskops 10 |
| 14 | Schaft des Endoskops 10 |
| 15 | Arbeitskanal des Endoskops 10 |
| 16 | Stirnseite des Schafts 14 |
| 17 | Endabschnitt |
| 18 | Beleuchtungseinrichtung |
| 19 | erste Lichtquelle |
| 20 | zweite Lichtquelle |
| 21 | dritte Lichtquelle |
| 22 | Lichtaufnahmeeinrichtung |
| 23 | Bildsensor |
| R | rotempfindliches Pixel |
| G | grünempfindliches Pixel |
| B | blauempfindliches Pixel |
| IR | infrarotempfindliches Pixel |
| 24 | erste optische Einrichtung |
| 25 | zweite optische Einrichtung |
| 251, 252 | Teile der zweiten optischen Einrichtung |
| 26 | dritte optische Einrichtung |
| 261, 262 | Teile der dritten optischen Einrichtung |
| 27 | Objektiv der optischen Einrichtung 24 |
| 28 | Linse des Objektivs 27 |
| 29 | erste Zone (Bildgebungszone) |
| 30 | Schmalseite |
| 31 | zweite Zone (Messzone) |
| 311, 312 | Teilzonen der zweiten Zone 31 |
| 32 | Gewebeoberfläche |
| 33 | dritte Zone (Messzone) |
| 331, 332 | Teilzonen der dritten Zone 31 |
| 333, 334 | Farbfilter |
| F1 | erster Multibandpassfilter |
| F2 | zweiter Multibandpassfilter |
| a - h | Durchlassbänder der Filter F1, F2 |
| 34 | vierte Zone (Messzone) |
| 35 | Objektiv |

**Patentansprüche**

1.   Endoskop (10) zur medizinischen Untersuchung und/oder Behandlung eines menschlichen oder tierischen Körpers,

mit einem länglichen Schaft (14), der an seinem distalen Ende (11) eine Beleuchtungseinrichtung (18) und eine Lichtaufnahmeeinrichtung (22) aufweist,
**dadurch gekennzeichnet,**
**dass** die Lichtaufnahmeeinrichtung (22) mindestens eine abbildende erste optische Einrichtung (24) und eine zweite optische Einrichtung (25) umfassen, denen eine erste Zone (29) und eine zweite Zone (31) eines gemeinsamen Bildsensors (23) zugeordnet sind, um auf dem Bildsensor (23) eine Bildgebungszone (29) und mindestens eine von dieser verschiedene Messzone (31) festzulegen.

2.   Endoskop nach Anspruch 1, **dadurch gekennzeichnet, dass** die erste optische Einrichtung (24) und die zweite optische Einrichtung (25) jeweils eine optische Achse festlegend ausgebildet sind

3.   Endoskop nach Anspruch 2, **dadurch gekennzeichnet, dass** diese beiden optischen Achsen parallel zueinander festgelegt sind.

4.  Endoskop nach einem der vorstehenden Ansprüche, **dadurch gekennzeichnet, dass** die Lichtaufnahmeeinrichtung (22) eine dritte optische Einrichtung (26) aufweist, der auf dem Bildsensor (23) wenigstens eine dritte Zone (33) zugeordnet ist, die eine weitere von der Bildgebungszone (29) verschiedene Messzone bildet.

5.  Endoskop nach einem der vorstehenden Ansprüche, **dadurch gekennzeichnet, dass** die zweite optische Einrichtung (25) zwei Lichteintrittsfenster (251, 252) aufweist, denen unterschiedliche zweite Zonen (311, 312) des Bildsensors (23) zugeordnet sind,

6.  Endoskop nach Anspruch 5, **dadurch gekennzeichnet, dass** die beiden Lichteintrittsfenster (251, 252) in unterschiedlichen Abständen zu der Beleuchtungseinrichtung (18) angeordnet sind.

7.  Endoskop nach einem der vorstehenden Ansprüche, **dadurch gekennzeichnet, dass** Beleuchtungseinrichtung (18) wenigstens eine Lichtquelle (19, 20, 21) mit einer Lichtaustrittsrichtung (A2) umfasst, die parallel zu der optischen Achse (A1) der ersten optischen Einrichtung (24) festgelegt ist, wobei die Beleuchtungseinrichtung (18) wenigstens eine Lichtquelle (21) umfasst, die in einem seitlichen Abstand zu der ersten optischen Einrichtung (24) angeordnet ist.

8.  Endoskop nach einem der vorstehenden Ansprüche, **dadurch gekennzeichnet, dass** die Bildgebungszone (29) zwischen zwei Messzonen (31/34; 331, 332) angeordnet ist.

9.  Endoskop nach einem der vorstehenden Ansprüche, **dadurch gekennzeichnet, dass** die zweite optische Einrichtung (25) zur Durchführung einer spektrometrischen Messung und/oder zur Durchführung einer differenzoptischen Messung eingerichtet ist.

10. Endoskop nach einem der vorstehenden Ansprüche, **dadurch gekennzeichnet, dass** die zweite optische Einrichtung (25) bezüglich der ersten optischen Einrichtung (24) auf dem Bildsensor (23) asymmetrisch angeordnete Teilbereiche (251, 252) nutzend angeordnet ist und/oder dass eine dritte optische Einrichtung (26) bezüglich der ersten optischen Einrichtung (24) auf dem Bildsensor (23) symmetrisch angeordnete Teilbereiche (261, 262) nutzend angeordnet ist.

11. Endoskop nach einem der Ansprüche 9 oder 10, **dadurch gekennzeichnet, dass** auf dem Bildsensor (23) wenigstens zwei auf unterschiedliche Wellenlängen abgestimmte Farbfilter (333, 334, F1, F2) aufgebracht sind, um ein Spektrometer zu bilden.

12. Endoskop nach Anspruch 11, **dadurch gekennzeichnet, dass** der Bildsensor (23) mehrere vorzugsweise in einem periodischen Raster angeordnete, für verschiedene Wellenlängenbereiche empfindliche Pixel (R, G, B, IR) aufweist und dass die Farbfilter (F1, F2) Multibandpassfilter mit jeweils mehreren Durchlassbändern (a, d, e, h; b, c, f, g) sind.

13. Endoskop nach Anspruch 12, **dadurch gekennzeichnet, dass** wenigstens zwei der Wellenlängenbereiche der verschiedenen Pixel (R, G, B, IR) einander überlappen, so dass Licht mit einer Wellenlänge im Überlappungsbereich in beiden überlappend empfindlichen Pixel Signale erzeugt.

14. Endoskop nach Anspruch 12 oder 13, **dadurch gekennzeichnet, dass** wenigstens eines der Multibandpassfilter (F1, F2) wenigstens ein Durchlassband (e, c) aufweist, das zwischen benachbarten Wellenlängenbereichen entsprechender Pixel (R, G, B, IR) liegt.

15. Endoskop nach Anspruch 11 bis 14, **dadurch gekennzeichnet, dass** die als dass die als Multibandpassfilter ausgebildeten Farbfilter (F1, F2) jeweils eine Gruppe mehrerer für verschiedene Wellenlängenbereiche empfindliche Pixel (R, G, B, IR) überdeckend angeordnet sind, wobei jede Gruppe jeweils mehrere Pixel (R, G, B, IR) jedes Wellenlängenbereichs enthält.

Fig. 1

Fig. 2

Fig. 3

Fig. 5

Fig. 4

A1

32

27    24    A2

28

29

21    17

23

Fig. 6

31

311 312

F1 } 34
F2

29

331 332 Fig. 7

33

23

Fig. 8

I

a     d e          h

F1

B    G     R    IR   λ    Fig. 9

I

b c        f      g

F2

B    G     R    IR   λ    Fig. 10

a b c d e f      g h

λ    Fig. 11

Europäisches
Patentamt

European
Patent Office

Office européen
des brevets

# EUROPÄISCHER RECHERCHENBERICHT

**Nummer der Anmeldung**

EP 24 21 0937

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2014 013594 A1 (VIIMAGIC GMBH [DE]) 28. Mai 2015 (2015-05-28) | 1,7 | INV.<br>A61B1/00 |
| Y | * Absätze [0058], [0059], [0061], [0062] *<br>* Abbildungen 4,5 *<br>- - - - - | 9 | A61B1/05<br>A61B1/06<br>A61B5/00 |
| X | US 2022/244462 A1 (DUCKETT III GEORGE E [US]) 4. August 2022 (2022-08-04) | 1-3 | |
| Y | * Absätze [0038], [0042] - [0044] *<br>* Absätze [0046], [0061] *<br>* Abbildungen 1, 2, 10 *<br>- - - - - | 9 | |
| X | US 2015/334374 A1 (YUN ZHISHENG [US] ET AL) 19. November 2015 (2015-11-19) | 1,4,10 | |
| Y | * Absätze [0001], [0021], [0027] *<br>* Absätze [0028], [0030], [0031] *<br>* Abbildungen 1, 4, 7, 9, 13, 14 *<br>- - - - - | 9 | |
| X | US 2019/231220 A1 (REFAI HAKKI [US] ET AL) 1. August 2019 (2019-08-01) | 1-8 | |
| Y | * Absätze [0041], [0046], [0051] *<br>* Absätze [0065], [0068], [0078] *<br>* Absätze [0120], [0139] *<br>* Abbildungen 1A, 2, 3, 10, 11 *<br>- - - - - | 9,11-15 | RECHERCHIERTE SACHGEBIETE (IPC)<br><br>A61B<br>G02B<br>H04N |
| Y | EP 2 226 648 A2 (FRAUNHOFER GES FORSCHUNG [DE]) 8. September 2010 (2010-09-08)<br>* Absätze [0040], [0073] - [0078], [0084] *<br>* Abbildungen 4A,4C,14 *<br>- - - - - | 9,11-15 | |

Der vorliegende Recherchenbericht wurde für alle Patentansprüche erstellt

| Recherchenort | Abschlußdatum der Recherche | Prüfer |
|---|---|---|
| München | 7. März 2025 | Neumann, Wiebke |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...............................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**          EP 24 21 0937

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-03-2025

| Im Recherchenbericht angeführtes Patentdokument | Datum der Veröffentlichung | Mitglied(er) der Patentfamilie | Datum der Veröffentlichung |
|---|---|---|---|
| DE 102014013594 A1 | 28-05-2015 | KEINE | |
| US 2022244462 A1 | 04-08-2022 | EP 3824792 A1 | 26-05-2021 |
| | | US 2021157057 A1 | 27-05-2021 |
| | | US 2022244462 A1 | 04-08-2022 |
| US 2015334374 A1 | 19-11-2015 | EP 3143453 A1 | 22-03-2017 |
| | | US 2015334374 A1 | 19-11-2015 |
| | | US 2017127042 A1 | 04-05-2017 |
| | | WO 2015175205 A1 | 19-11-2015 |
| US 2019231220 A1 | 01-08-2019 | US 2019231220 A1 | 01-08-2019 |
| | | US 2023380659 A1 | 30-11-2023 |
| | | WO 2019104329 A1 | 31-05-2019 |
| EP 2226648 A2 | 08-09-2010 | DE 102008011793 A1 | 24-09-2009 |
| | | EP 2208033 A2 | 21-07-2010 |
| | | EP 2226648 A2 | 08-09-2010 |
| | | WO 2009106316 A2 | 03-09-2009 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2075617 A1
- EP 2725967 B1
- EP 2284509 A1
- WO 2018072806 A1
- EP 0939894 B1
- WO 2010129324 A
- US 20050154277 A1
- US 20090147076 A1
- US 20100141380 A1

**In der Beschreibung aufgeführte Nicht-Patentliteratur**

- **YUHANG WAN** ; **SAOUD A. AL-MULLA** ; **WANG PENG** ; **KENNETH D. LONG** ; **BENJAMIN A. KESLER** ; **PATRICK SU** ; **JOHN M. DALLESASSE** ; **BRIAN T. CUNNIGHAM**. *Integrated spectroscopic analysis system with low vertical height for measuring liquid or solid assays*, http://www.elsevier.com/open-access/userlicense/1.0/